# EUROPEAN PATENT SPECIFICATION

(11) **EP 1 523 958 B1**
(45) Date of publication and mention of the grant of the patent: **01.08.2012**
(21) Application number: 04256298.3
(22) Date of filing: 13.10.2004
(51) Int. Cl.: A61F 2/06

(54) **Graft coupling apparatus and methods of using same**
Gefässprothesenkupplungsvorrichtung und Verfahren zu deren Verwendung
Dispositif de connexion de prothèse vasculaire et méthode d'utilisation

(30) Priority: 14.10.2003 US 685374
(43) Date of publication of application: 20.04.2005
(73) Proprietor: Cordis Corporation, Miami Lakes, Florida 33014 (US)
(72) Inventor: Bonsignore, Craig, San José, CA 95112 (US)
(74) Representative: Belcher, Simon James

(56) References cited:
- US-A- 5 984 955
- US-A1- 2002 099 441
- US-A1- 2002 173 808
- US-A1- 2004 073 282
- US-A1- 2004 116 945

## Description

The present invention relates to medical devices and methods for joining two vessels. More particularly, the present invention relates to devices and methods for using grafts, stents and other endovascular devices as a coupling device for joining two vessels.

Endovascular grafts, stents and like devices are known in the art for aiding, repairing or bypassing blood flow through vascular vessels. As will be appreciated, these prior art devices require skill and precision during surgery to deliver and properly couple the device to the vasculature of the patient. Take bypass grafts for example. Aside from the difficulties associated with delivering the graft to the proper location, heretofore, in typical femoral-femoral or femoral-popliteal bypasses, for example, the bypass graft must be manually sutured to the native vessel. Surgically suturing the graft is a tedious and time consuming process, requiring substantial skill and experience to achieve a secure and leak-free coupling. Undesirable results, such as leaks, are not uncommon, and require the suturing to be modified or supplemented. The adverse consequences posed by suturing are clear drawbacks with these prior art grafts and like devices.

US-5984955 discusses a system for endoluminal grafting of a main conduit to a branch conduit. It mentions a branch graft connector apparatus that is incorporated into the primary or branch graft. It comprises two parts, a first part attached to a primary graft comprises a ring surrounding the branch graft opening and a fructoconical member extending outwardly from the ring. The second part attached to a branch graft comprises a tapered portion and two ring members spaced apart from each other and formed on the tapered portion. The two parts abut each other to join the primary and branch grafts.

US-A-2002/0173880 relates to sutureless anastomosis systems. It mentions securing portions of vessel walls to fittings using rings.

Accordingly, there exists a long-felt, yet unresolved need in the art for improved devices and methods for endovascular treatment and repairs, such as coupling grafts and bypass grafts and the like to native vessels, without the need for suturing.

The present invention overcomes the practical problems described above and offers new advantages as well. Yet another object of the present invention is to provide devices for coupling vasculature end-to-side.

These and other objects and advantages of the present invention may be realized by an artificial graft coupling device including internal and external anchors to seal the endovascular vessel and graft.

One advantageous feature of the present invention is the provision of devices for end-to-side coupling of an artificial graft and/or donor vessel to a main vessel.

In accordance with an aspect of the present invention, a device for coupling a bypass vessel to the side of a native vessel is provided as defined in appended claim 1. In another aspect of the invention, a device for coupling a bypass vessel to the side of a native vessel is provided as defined in appended claim 3. The devices of both aspects comprise a main trunk, first and second stent-anchors associated with the main trunk and a graft extension extending from said main trunk. An internal anchor and an external anchor cooperate to seal said graft extension and the bypass vessel.

The device of the invention can be used in a method not part of the invention of performing end-to-side anastamosis comprising the steps of:
inserting a graft coupling device having a main trunk and a graft extension in a main vessel having an incision such that said graft extension protrudes from said extension;
sealing said main trunk to said main vessel with anchors;
placing a bypass graft over a portion of said graft extension; and
sealing said bypass graft and said graft extension with cooperating interior and exterior anchors.

The device of the invention can also be used in a method not part of the invention of performing end-to-side anastamosis comprising the steps of:
delivering a first guide wire to a junction site in a main vessel;
puncturing said main vessel at said junction site;
delivering a graft coupling device having a main trunk and a graft extension having an external anchor to said junction site via said first guide wire;
positioning a second guide wire to exit said graft extension of said graft coupling device at said puncture site;
sealing said main trunk to said main vessel with interior anchors disposed on opposite ends of said puncture site;
advancing a balloon along said second guide wire to a location corresponding to said external anchor;
balloon expanding said external anchor to a diameter sufficient for accepting a bypass graft;
navigating a bypass graft to said junction site and into said graft extension;
advancing an internal anchor along said second guide wire; and
deploying said internal anchor, thereby sealing said extension graft and said bypass graft.

The present invention is based, in part, on the discovery that internal and external stent-like structures may be adapted to serve as anchors and seals to facilitate sutureless coupling of two or more vessels. While the present invention will be described in connection with end-to-side anastamosis methods and devices, one of ordinary skill in the art will readily appreciate that the present invention may be adapted for numerous uses in a variety of fields.

Embodiments of the invention will now be described by way of example with reference to the accompanying drawings, in which:
Figure 1 is a plan view of an embodiment of a graft coupling device for end-to-side sutureless coupling of vessels according to the invention; and
Figure 2 is a plan view of another embodiment of a graft coupling device for end-to-side sutureless coupling of vessels according to the invention;

Like reference numbers indicate identical or functionally similar elements.

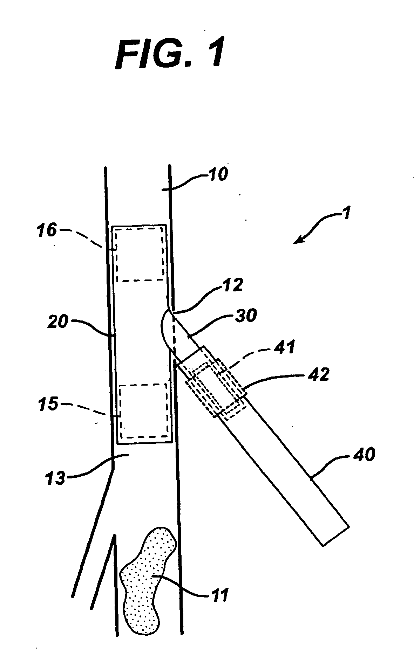

Figure 1 depicts a presently preferred embodiment of a graft coupling device 1 according to the invention for end-to-side anastamosis. As depicted in FIG.1, native vessel 10 has some disease or obstruction 11, requiring a bypass graft to preserve flow to some distal point in the vasculature. Such a situation is typical of femoral-femoral or femoral-politeal bypass procedures. As will be appreciated, in the case of end-to-side anastamosis, it is considered favorable to maintain flow distal to the site of graft attachment. In such a procedure, native vessel 10 will be accessed in an open procedure to allow a surgeon to make an incision 12 in side wall of vessel 10. With direct access to vessel 10 available, the surgeon places main trunk 20 of graft coupling device 1 in the interior 13 of vessel 10. In placing the device 1, the surgeon allows graft extension 30 to protrude from the incision 12.

Proximal and distal main trunk anchors 15, 16 serve to seal main trunk 20 to native vessel 10 proximally and distally to the incision 12 and graft extension 30. Anchors 15, 16 may be integral to main trunk 20, or alternatively, may be deployed in a secondary step. Preferably, anchors 15, 16 are interior anchors which expand radially or otherwise exert an outward force to frictionally seal main trunk 20 in place. Alternatively, exterior anchors which constrict radially or otherwise exert an inward force may be used.

Bypass graft 40 is extended over coupling graft extension 30. Preferably, graft extension 30 is sized such that its diameter approximates the inner diameter of bypass graft 40. An internal graft anchor 41 within bypass graft 40 exerts an outward force, while an external graft anchor 42 exerts and inward force. The opposing forces from these anchors secure the seal between the graft extension 30 and bypass graft 40. The opposing forces of using both anchors is preferable in sealing and ensuring the structural integrity of the device and its positioning is maintained.

The anchors according to the invention may be constructed of any suitable material and shaped in any suitable configuration. Preferably, the internal and external anchors exhibit stent-like characteristics. The internal and external stent-like anchors may both feature superelastic properties, or alternatively one or both may be plastically deformable.

In the case of a plastically deforming internal stent coupled with a superelastic external stent, the sealing pressure could be adjusted by balloon dilation of the internal stent. As the internal stent is ratcheted up in diameter, it increases the diameter of the external superelastic stent, thereby increasing the inward force it exerts on the graft-to-vessel seal.

In the case of a plastically deformable external stent, a similar effect may be achieved using a balloon internally to force expansion of the graft, vessel, and both anchor stents. Alternatively, the deformable external stent may be manually crimped or constrained in diameter to further reengage the superelastic internal stent, also thereby increasing sealing pressure.

In the cases wherein both the external and internal anchors are superelastic, their diameters would be set such that they would engage each other over a range of diameters with predictable or predetermined resultant sealing pressure. As such, the memory diameter of the external anchor would be smaller than the anticipated outer diameter of the external graft or vessel (depending on the procedure), while the memory diameter of the internal anchor would be larger than the anticipated inner diameter of the internal graft or vessel.

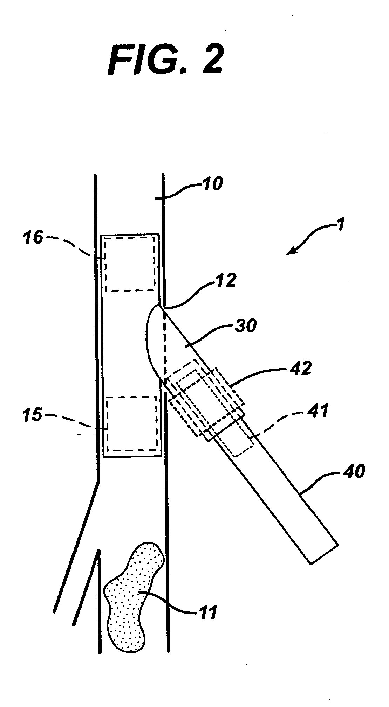

In an alternative exemplary embodiment for end-to-side anastamosis depicted in Figure 2, the roles of the graft extension 30 and bypass graft 40 are reversed such that the graft extension is external and the bypass graft is internal. In accordance with this exemplary embodiment, the external stent-like anchor 42 could be integral with the graft extension 30 as an exoskeleton. This exemplary embodiment offers the possibility of a less invasive procedure in that the device and coupling can be achieved without external access to the vessel or junction site.

In operation, access to the femoral artery 10 (or other target vessel) is achieved through normal means well known in the art of endovascular procedures. A guide wire (not shown) is delivered to the site at which the bypass graft 40 is to join the main vessel 10. Once properly positioned, blood flow to the region is attenuated by some other endovascular means, for example, deployment of a balloon proximal to the site, preferably proximal to the wire access point. The desired junction site is deliberately punctured and dilated using endovascular techniques within the skill of the ordinary artisan. The graft coupling device 1, including main trunk 20, graft extension 30, and internal anchors 15, 16 and 41, is loaded in a constrained state into a delivery device (not shown). The delivery device is advanced along the guide wire to the desired site. Preferably, the delivery system allows positioning of a second guidewire which exits the graft extension 30.

The primary guidewire remains in the main vessel, while the secondary guidewire is navigated to exit the vessel 10 at the puncture site. Anchors 15, 16 are deployed to seal main trunk 20 of the coupling graft device 1 into the main vessel 10. The external anchor exoskeleton 42 in this exemplary embodiment is preferably plastically deformable. Accordingly, at this point in the procedure, a balloon is advanced along the secondary guidewire to the location of external anchor 42, and it is partially expanded to a diameter which allows it to accommodate bypass graft 40.

Bypass graft 40 is preferably navigated to the attachment site using some endoscopic other minimally invasive technique. Preferably, the device used to advance the graft must have the capability to locate and snare the second guidewire which was punctured through the vessel as described above. Once the guidewire is successfully located, the bypass graft 40 may be advanced into the graft extension 30 (which has preferably been partially expanded as previously described). Internal anchor 41 may now be advanced along the secondary guidewire and deployed inside bypass graft 40.

A final balloon inflation inside the internal anchor 41 fully expands external anchor 42 and fully engages the internal and external anchors to provide an adequate seal. Accordingly, at this point the vessels have been joined together without external manual access to the junction site. Blood flow may be restored when the opposite end of the bypass graft is properly terminated.

## Claims

1. A device for coupling a bypass vessel (40) to the side of a native vessel (10) comprising:
a main trunk (20);
first and second stent-anchors (15, 16) associated with said main trunk (20); and
a graft extension (30) extending from said main trunk (20);
**characterised in that** the device further comprises
an internal stent-like anchor (41) and an external stent-like anchor (42); wherein in use said bypass vessel (40) extends over said graft extension (30), said internal anchor (41) is disposed in said graft extension (30) in an area at least partially overlapped by said bypass vessel (40) and said external anchor (42) is disposed outside said bypass vessel (40) such that said internal anchor (41) and said external anchor (42) cooperate to seal said graft extension (30) and said bypass vessel (40).

2. The device of claim 1, wherein said external anchor (42) is integral with said bypass vessel (40) in an area which at least partially overlaps said bypass vessel (40).

3. A device for coupling a bypass vessel (40) to the side of a native vessel (10) comprising:
a main trunk (20);
first and second stent-anchors (15, 16) associated with said main trunk (20); and
a graft extension (30) extending from said main trunk (20);
**characterised in that** the device further comprises
an internal stent-like anchor (41) and an external stent-like anchor (42); wherein in use said graft extension (30) extends over said bypass vessel (40), said internal anchor (41) is disposed in said bypass vessel (40) in an area at least partially overlapped by said graft extension (30) and said external anchor (42) is disposed outside said graft extension (30) such that said internal anchor (41) and said external anchor (42) cooperate to seal said graft extension (30) and said bypass vessel (40).

4. The device of claim 3 wherein said external anchor (42) is integral with said graft extension (30) in an area which at least partially overlaps said bypass vessel (40).

5. The device of any one of the preceding claims further comprising a bypass vessel (40) which comprises an artificial graft.

6. The device of claim 1 or 2 wherein said first and second stent-anchors (15, 16) are disposed in an interior area of said main trunk (20) on opposite ends of said graft extension (30).

7. The device of claim 6 wherein said first and second stent-anchors (15, 16) exude an outward force to seal said main trunk (20) in the native vessel (10).

8. The device of claim 7 wherein said first and/or second stent-anchors (15, 16) comprise a plastically deformable material.

## Patentansprüche

1. Vorrichtung zum Koppeln eines Bypassgefäßes (40) an die Seite eines natürlichen Gefäßes (10), welche Folgendes umfasst:
einen Hauptgefäßstrang (20);
erste und zweite Stent-Anker (15, 16), die mit dem Hauptgefäßstrang (20) verknüpft sind; und
eine Gefäßprothesenerweiterung (30), die sich von dem Hauptgefäßstrang (20) erstreckt,
**dadurch gekennzeichnet, dass** die Vorrichtung ferner Folgendes umfasst:
einen internen stentartigen Anker (41) und einen externen stentartigen Anker (42); wobei sich das Bypassgefäß (40) im Gebrauch über die Gefäßprothesenerweiterung (30) erstreckt, der interne Anker (41) in der Gefäßprothesenerweiterung (30) in einem Bereich angeordnet ist, der sich wenigstens teilweise mit dem Bypassgefäß (40) überlappt, und der externe Anker (42) außerhalb des Bypassgefäßes (40) angeordnet ist, so dass der interne Anker (41) und der externe Anker (42) zusammenwirken, um die Gefäßprothesenerweiterung (30) und das Bypassgefäß (40) abzudichten.

2. Vorrichtung nach Anspruch 1, wobei der externe Anker (42) an dem Bypassgefäß (40) in einem Bereich integriert ist, der sich wenigstens teilweise mit Bypassgefäß (40) überlappt.

3. Vorrichtung zum Koppeln eines Bypassgefäßes (40) an die Seite eines natürlichen Gefäßes (10), welche Folgendes umfasst:
einen Hauptgefäßstrang (20);
erste und zweite Stent-Anker (15, 16), die mit dem Hauptgefäßstrang (20) verknüpft sind; und
eine Gefäßprothesenerweiterung (30), die sich von dem Hauptgefäßstrang (20) erstreckt,
**dadurch gekennzeichnet, dass** die Vorrichtung ferner Folgendes umfasst:
einen internen stentartigen Anker (41) und einen externen stentartigen Anker (42); wobei sich die Gefäßprothesenerweiterung (30) im Gebrauch über das Bypassgefäß (40) erstreckt, der interne Anker (41) in dem Bypassgefäß (40) in einem Bereich angeordnet ist, der sich wenigstens teilweise mit der Gefäßprothesenerweiterung (30) überlappt, und der externe Anker (42) außerhalb der Gefäßprothesenerweiterung (30) angeordnet ist, so dass der interne Anker (41) und der externe Anker (42) zusammenwirken, um die Gefäßprothesenerweiterung (30) und das Bypassgefäß (40) abzudichten.

4. Gefäß nach Anspruch 3, wobei der externe Anker (42) an der Gefäßprothesenerweiterung (30) in einem Bereich integriert ist, der sich wenigstens teilweise mit dem Bypassgefäß (40) überlappt.

5. Vorrichtung nach einem der vorhergehenden Ansprüche, das weiterhin ein Bypassgefäß (40) umfasst, welches eine künstliche Gefäßprothese umfasst.

6. Vorrichtung nach Anspruch 1 oder 2, wobei der erste und der zweite Stent-Anker (15, 16) in einen Innenbereich des Hauptgefäßstrangs (20) an gegenüberliegenden Enden der Gefäßprothesenerweiterung (30) angeordnet sind.

7. Vorrichtung nach Anspruch 6, wobei der erste und der zweite Stent-Anker (15, 16) eine nach außen gerichtete Kraft aufbringen, um den Hauptgefäßstrang (20) in dem natürlichen Gefäß (10) abzudichten.

8. Vorrichtung nach Anspruch 7, wobei der erste und/oder der zweite Stent-Anker (15, 16) ein platisch deformierbares Material umfasst/umfassen.

## Revendications

1. Dispositif destiné à coupler un vaisseau de dérivation (40) au côté d'un vaisseau naturel (10) comprenant :
un tronc principal (20) ;
des première et secondes ancres d'endoprothèse vasculaire (15, 16) associées audit tronc principal (20) ; et
une extension de greffon (30) qui s'étend à partir dudit tronc principal (20) ;
**caractérisé en ce que** le dispositif comprend en outre :
une ancre intérieure similaire à une endoprothèse vasculaire (41) et une ancre extérieure similaire à une endoprothèse vasculaire (42) ; dans lequel, en service, ledit vaisseau de dérivation (40) s'étend au-dessus de ladite extension de greffon (30), ladite ancre intérieure (41) est disposée dans ladite extension de greffon (30) dans une zone recouverte au moins en partie par ledit vaisseau de dérivation (40) et ladite ancre extérieure (42) est disposée à l'extérieur dudit vaisseau de dérivation (40) de telle sorte que ladite ancre intérieure (41) et ladite ancre extérieure (42) coopèrent de façon à sceller ladite extension de greffon (30) et ledit vaisseau de dérivation (40).

2. Dispositif selon la revendication 1, dans lequel ladite ancre extérieure (42) est formée d'une pièce avec ledit vaisseau de dérivation (40) dans une zone qui recouvre au moins en partie ledit vaisseau de dérivation (40).

3. Dispositif destiné à coupler un vaisseau de dérivation (40) au côté d'un vaisseau naturel (10) comprenant :
un tronc principal (20) ;
des première et secondes ancres d'endoprothèse vasculaire (15, 16) associées audit tronc principal (20) ; et
une extension de greffon (30) qui s'étend à partir dudit tronc principal (20) ;
**caractérisé en ce que** le dispositif comprend en outre :
une ancre intérieure similaire à une endoprothèse vasculaire (41) et une ancre extérieure similaire à une endoprothèse vasculaire (42) ; dans lequel, en service, ladite extension de greffon (30) s'étend au-dessus dudit vaisseau de dérivation (40), ladite ancre intérieure (41) est disposée dans ledit vaisseau de dérivation (40) dans une zone recouverte au moins en partie par ladite extension de greffon (30) et ladite ancre extérieure (42) est disposée à l'extérieur de ladite extension de greffon (30) de telle sorte que ladite ancre intérieure (41) et ladite ancre extérieure (42) coopèrent de façon à sceller ladite extension de greffon (30) et ledit vaisseau de dérivation (40).

4. Dispositif selon la revendication 3, dans lequel ladite ancre extérieure (42) est formée d'une pièce avec ladite extension de greffon (30) dans une zone qui recouvre au moins en partie ledit vaisseau de dérivation (40).

5. Dispositif selon l'une quelconque des revendications précédentes, comprenant en outre un vaisseau de dérivation (40) qui comprend un greffon artificiel.

6. Dispositif selon la revendication 1 ou la revendication 2, dans lequel lesdites première et seconde ancres similaires à une endoprothèse vasculaire (15, 16) sont disposées dans une zone intérieure dudit tronc principal (20) sur des extrémités opposées de ladite extension de greffon (30).

7. Dispositif selon la revendication 6, dans lequel lesdites première et seconde ancres similaires à une endoprothèse vasculaire (15, 16) exercent une force vers l'extérieur de façon à sceller ledit tronc principal (20) dans le vaisseau natif (10).

8. Dispositif selon la revendication 7, dans lequel lesdites première et/ou secondes ancres similaires à une endoprothèse vasculaire (15, 16) comprennent un matériau déformable de manière plastique.
